# EUROPEAN PATENT APPLICATION

(11) **EP 1 400 594 A1**
(43) Date of publication of application: **24.03.2004**
(21) Application number: 02738795.0
(22) Date of filing: 25.06.2002
(51) Int. Cl.: C12P 7/02

(54) **PROCESS FOR PRODUCING OPTICALLY ACTIVE (R)-2-CHLORO-1-(3'-CHLOROPHENYL)ETHA NOL**

(30) Priority: 25.06.2001 JP 2001191517
(71) Applicant: KANEKA CORPORATION, Osaka-shi, Osaka 530-8288 (JP)
(72) Inventor: SHIMIZU, Sakayu, Kyoto-shi, Kyoto 616-8212 (JP); KATAOKA, Michihiko, Kyoto-shi, Kyoto 606-0082 (JP); KIZAKI, Noriyuki, c/o KANEKA CORPORATION, Takasago-shi, Hyogo 676-0027 (JP); YASOHARA, Yoshihiko, c/o KANEKA CORPORATION, Takasago-shi, Hyogo 676-0027 (JP)
(74) Representative: VOSSIUS & PARTNER
(86) International application number: PCT/JP2002/006343
(87) International publication number: WO 2003/000911

(57) **Abstract**

The present invention discloses a process for efficiently producing, on an industrial scale, optically active (R)-2-chloro-1-(3'-chlorophenyl)ethanol which is useful as a raw material for the synthesis of medicines, agricultural chemicals, etc.

The process includes allowing cells of a microorganism or a material prepared by treating the cells of the microorganism to act on 2-chloro-1-(3'-chlorophenyl)ethanone to produce (R)-2-chloro-1-(3'-chlorophenyl)ethanol, the microorganism being capable of stereoselectively reducing 2-chloro-1-(3'-chlorophenyl)ethanone to yield (R)-2-chloro-1-(3'-chlorophenyl)ethanol.

## Description

### Technical Field

The present invention relates to a process for producing optically active (R)-2-chloro-1-(3'-chlorophenyl)ethanol. Optically active (R)-2-chloro-1-(3'-chlorophenyl)ethanol is a compound useful as a raw material for the synthesis of medicines, agricultural chemicals, etc.

### Background Art

Processes for producing optically active (R)-2-chloro-1-(3'-chlorophenyl)ethanol are disclosed in JP-A-04-218384 and JP-A-11-215995, in which a microorganism belonging to the genus Ashbya, the genus Ogataea, or the like, or a substance prepared by treating the microorganism is allowed to act on 2-chloro-1-(3'-chlorophenyl)ethanone. However, in these processes, attainable substrate concentration is not sufficiently high in the reaction. Therefore, development of a more efficient process has been desired.

### Disclosure of Invention

It is an object of the present invention to provide a process for producing optically active (R)-2-chloro-1-(3'-chlorophenyl)ethanol efficiently on an industrial scale.

The present inventors have conducted intensive research in order to solve the problem described above and have discovered a novel enzyme source which is capable of stereoselectively reducing 2-chloro-1-(3'-chlorophenyl)ethanone to yield optically active (R)-2-chloro-1-(3'-chlorophenyl)ethanol, thus achieving the present invention.

That is, a process for producing (R)-2-chloro-1-(3'-chlorophenyl)ethanol of the present invention includes allowing a culture broth of a microorganism, cells of the microorganism, or a material prepared by treating the cells of the microorganism to act on 2-chloro-1-(3'-chlorophenyl)ethanone and collecting the resultant (R)-2-chloro-1-(3'-chlorophenyl)ethanol, the microorganism being capable of stereoselectively reducing 2-chloro-1-(3'-chlorophenyl)ethanone to yield (R)-2-chloro-1-(3'-chlorophenyl)ethanol and belonging to any one of the following genera: Escherichia, Aerobacter, Enterobacter, Klebsiella, Citrobacter, Rahnella, Erwinia, Serratia, Proteus, Morganella, Salmonella, Alcaligenes, Kocuria, Arthrobacter, Brevibacterium, Cellulomonas, Acinetobacter, Aeromonas, Bacillus, Agrobacterium, Nocardioides, Stenotrophomonas, Jensenia, Mycobacterium, Nocardia, Rhodococcus, Pseudonocardia, Streptomyces, Streptosporangium, Rothia, Williopsis, Kuraishia, Citeromyces, Saccharomycodes, Sporobolomyces, Dipodascus, Saccharomycopsis, Sporidiobolus, Zygosaccharomyces, Hyphopichia, Penicillium, Exophiala, Sporotrichum, Acremonium, Paecilomyces, Verticillium, Tilachlidium, Pitomyces, Monosporium, Isaria, Gloeophyllum, Strobilurus, and Crinipellis.

### Detailed Disclosure of the Invention

The present invention will be described in detail below.

In the present invention, 2-chloro-1-(3'-chlorophenyl)ethanone and (R)-2-chloro-1-(3'-chlorophenyl)ethanol are compounds represented by formula (1) and formula (2) below, respectively.

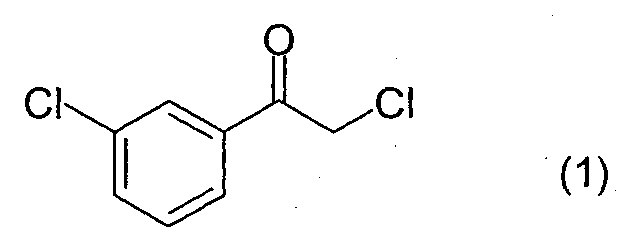

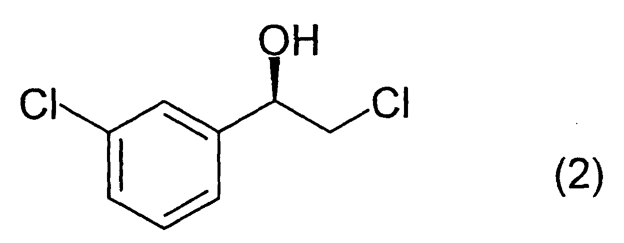

Additionally, in the following description, "%" means %(W/V) unless stated otherwise.

The microorganism, which is used in the present invention, capable of stereoselectively reducing 2-chloro-1-(3'-chlorophenyl)ethanone to yield (R)-2-chloro-1-(3'-chlorophenyl)ethanol can be found by the method described below.

For example, 50 ml of a culture medium (pH 7.0) comprising 1% of polypeptone, 1% of meat extract, 0.5% of yeast extract, and 0.3% of sodium chloride is placed into a 500-ml Sakaguchi flask, and sterilization is performed. The culture medium is inoculated with a microorganism and the flask is shaken at 30°C for 1 to 3 days. The grown cells are then collected by centrifugation and suspended in 5 ml of a phosphate buffer (pH 6.5) containing 0.1% to 0.5% of 2-chloro-1-(3'-chlorophenyl)ethanone and 5% of glucose. The resultant suspension is shaken in a test tube plugged with cotton for 1 to 3 days at 30°C.

In the method described above, the cells obtained by centrifugation may be dried in a desiccator or using acetone before use. Furthermore, when the microorganism or a material prepared by treating the microorganism is reacted with 2-chloro-1-(3'-chlorophenyl)ethanone, oxidized nicotinamide adenine dinucleotide (NAD) and/or oxidized nicotinamide adenine dinucleotide phosphate (NADP) and a glucose dehydrogenase may be added thereinto.

After the reaction is completed, ethyl acetate in the same volume as that of the reaction mixture is added thereinto and extraction is performed. The resultant 2-chloro-1-(3'-chlorophenyl)ethanol is separated and analyzed by high performance liquid chromatography under the following conditions.

Column: Chiralcel OJ (4.6 x 250 mm) manufactured by Daicel Chemical Industries, Ltd.
Eluent: hexane/isopropanol = 39/1 (V/V)
Flow rate: 1 ml/min
Detection: 210 nm
Column temperature: room temperature
Elution time [2-chloro-1-(3'-chlorophenyl)ethanone: 28 min, (R)-2-chloro-1-(3'-chlorophenyl)ethanol: 37 min, (S)-2-chloro-1-(3'-chlorophenyl)ethanol: 45 min]

The microorganisms usable in the present invention belong to the genera Escherichia, Aerobacter, Enterobacter, Klebsiella, Citrobacter, Rahnella, Erwinia, Serratia, Proteus, Morganella, Salmonella, Alcaligenes, Kocuria, Arthrobacter, Brevibacterium, Cellulomonas, Acinetobacter, Aeromonas, Bacillus, Agrobacterium, Nocardioides, Stenotrophomonas, Jensenia, Mycobacterium, Nocardia, Rhodococcus, Pseudonocardia, Streptomyces, Streptosporangium, Rothia, Williopsis, Kuraishia, Citeromyces, Saccharomycodes, Sporobolomyces, Dipodascus, Saccharomycopsis, Sporidiobolus, Zygosaccharomyces, Hyphopichia, Penicillium, Exophiala, Sporotrichum, Acremonium, Paecilomyces, Verticillium, Tilachlidium, Pitomyces, Monosporium, Isaria, Gloeophyllum, Strobilurus, and Crinipellis.

Specific examples which may be used include Escherichia coli IFO3301, Escherichia coli IFO13965, Aerobacter aerogenes IFO3166, Enterobacter cloacae IFO3320, Klebsiella pneumoniae IFO3318, Klebsiella pneumoniae IFO12059, Citrobacter freundii IFO13547, Citrobacter freundii IFO12681, Rahnella aquatilis IF013544, Erwinia carotovora subsp. carotovora IFO3380, Serratia marcescens IF03054, Proteus mirabilis IFO3849, Morganella morganii subsp. morganii IF03848, Salmonella typhimurium IF012529, Salmonella typhimurium IFO13245, Alcaligenes faecalis IF013111, Alcaligenes xylosoxidans subsp. denitrificans IF012669, Kocuria rosea IFO3764, Arthrobacter pascens IF012139, Arthrobacter protophormiae IFO12128, Brevibacterium linens IF012141, Cellulomonas fimi IAM12107, Acinetobacter calcoaceticus IF012552, Aeromonas hydrophila subsp. hydrophila IF03820, Bacillus subtilis IF03009, Bacillus thuringiensis IF03951, Agrobacterium tumefaciens IFO12667, Nocardioides simplex IFO12679, Stenotrophomonas maltophilia IFO12690, Jensenia canicruria IFO13914, Mycobacterium smegmatis IF03154, Mycobacterium phlei IF03158, Nocardia globerula IFO13510, Nocardia carnea IFO14403, Rhodococcus rhodochrous IFO3338, Rhodococcus erythropolis IFO12320, Pseudonocardia autotrophica IFO12743, Streptomyces griseolus IFO3402, Streptomyces scabies IF03111, Streptomyces lactamdurans IF03305, Streptosporangium roseum IFO3776, Rothia dentocariosa IFO12531, Williopsis saturnus var. saturnus IF00125, Kuraishia capsulata IF00974, Citeromyces matritensis IF00954, Saccharomycodes ludwigii IFO1043, Sporobolomyces salmonicolor IFO1038, Sporobolomyces roseus IFO1106, Dipodascus armillariae IF00102, Dipodascus tetrasuperma CBS765.70, Saccharomycopsis capsularis IFO0672, Sporidiobolus johnsonii IF06903, Zygosaccharomyces rouxii IFO0493, Hyphopichia burtonii IFO0844, Penicillium chrysogenus IF04626, Penicillium oxalicum IFO5748, Penicillium rubrum IF06580, Penicillium lilacinum IFO5752, Exophiala mansonii IFO6880, Sporotrichum aurantiacum IFO9381, Acremonium butyri IFO8580, Paecilomyces carneus IFO8292, Paecilomyces carneus IF08293, Verticillium albo-atrum IFO9470, Verticillium dahliae IF09765, Verticillium psalliotae IF030619, Tilachlidium humicola IFO5696, Pitomyces chartarum ATCC26953, Monosporium bharatensis ATCC18967, Isaria japonica IFO30367, Gloeophyllum trabeum IF06509, Strobilurus stephanocystis IFO30194, and Crinipellis stipitaria IF030259.

These microorganisms can be generally obtained from stock strains which are easily available or purchasable. They may also be isolated from nature. Additionally, these microorganisms may be subjected to mutation to produce strains which have properties more advantageous to the reaction of the present invention.

Any source of nutrition may be used to culture these microorganisms as long as it can be assimilated by the microorganisms. Examples thereof include saccharides, such as glucose, sucrose, and maltose; organic acids, such as lactic acid, acetic acid, citric acid, and propionic acid; alcohols, such as ethanol and glycerin; hydrocarbons, such as paraffins; and oils and fats, such as soybean oil and colza oil. Alternatively, carbon sources, such as mixtures of these substances, and nitrogen sources, such as ammonium sulfate, ammonium phosphate, urea, yeast extracts, meat extracts, peptones, and corn steep liquor may be mixed. Furthermore, sources of nutrition, such as other inorganic salts and vitamins, may be added as appropriate.

The microorganisms can be cultured under common conditions. For example, culture is performed aerobically at a pH of 4.0 to 9.5, in a temperature range of 20°C to 45°C, for 10 to 96 hours.

When a microorganism is allowed to react with 2-chloro-1-(3'-chlorophenyl)ethanone, the culture solution of the microorganism may be used for the reaction as it is, or the concentrated culture solution may be used. When the components in the culture broth adversely affect the reaction, preferably, the cells of the microorganism obtained by centrifugation of the culture broth, or a material prepared by treating the cells of the microorganism are used.

The material prepared by treating the cells of the microorganism is not particularly limited. Examples thereof include dried cells obtained by dehydration with acetone or diphosphorus pentaoxide or by drying using a desiccator or fan, surfactant-treated materials, lytic enzyme-treated materials, immobilized cells, and cell-free extract samples prepared by cell disruption. These materials may be subjected to heat treatment before use. Furthermore, an enzyme catalyzing asymmetric reduction may be prepared by purification of a culture and used.

In the reduction reaction, 2-chloro-1-(3'-chlorophenyl)ethanone which is the substrate may be added at one time in the initial stage of the reaction or may be added in portions as the reaction proceeds.

The reaction temperature is usually 10°C to 60°C, and preferably 20°C to 40°C. The pH during the reaction is 2.5 to 9, and preferably 5 to 9.

The amount of the enzyme source in the reaction mixture may be set appropriately depending on the substrate-reducing ability. The substrate concentration in the reaction mixture is preferably 0.01% to 50%, and more preferably 0.1% to 30%.

The reaction is usually carried out while the reaction mixture is shaken or stirred under aeration. The reaction time is set appropriately depending on the substrate concentration, the amount of the enzyme source, and other reaction conditions. Preferably, the individual conditions are set so that the reaction is completed in 2 to 168 hours.

In order to accelerate the reduction reaction, preferably, an energy source, such as glucose or ethanol, is added into the reaction mixture in an amount of 1% to 30%. Consequently, excellent results are obtained. Additionally, the reaction may be accelerated by adding a coenzyme which is generally required in the reduction reaction by a biological method, such as a reduced-nicotinamide adenine dinucleotide (NADH) or reduced-nicotinamide adenine dinucleotide phosphate (NADPH). Specifically, these may be added directly into the reaction mixture, or a reaction system which generates NADH or NADPH may be added into the reaction mixture together with an oxidized-type coenzyme. For example, a reaction system in which a formate dehydrogenase reduces NAD to NADH when it produces carbon dioxide and water from formic acid, or a reaction system in which a glucose dehydrogenase reduces NAD or NADP to NADH or NADPH, respectively, when it produces gluconolactone from glucose may be used.

It is also effective to add a surfactant, such as Triton (manufactured by Nacalai Tesque, Inc.), Span (manufactured by Kanto Kagaku), or Tween (manufactured by Nacalai Tesque, Inc.), into the reaction mixture.

Furthermore, in order to prevent the reaction from being inhibited by the substrate and/or the alcohol which is the product of the reduction reaction, a water-insoluble organic solvent, such as ethyl acetate, n-butyl acetate, isopropyl ether, or toluene, may be added into the reaction mixture. In order to increase the solubility of the substrate, a water-soluble organic solvent, such as methanol, ethanol, acetone, tetrahydrofuran, or dimethyl sulfoxide, may also be added into the reaction mixture.

After the reduction reaction, the reaction mixture is directly, or with the cells of the microorganism, etc., being isolated, extracted with a solvent, such as ethyl acetate or n-hexane, and the solvent is removed from the extract. Thereby, (R)-2-chloro-1-(3'-chlorophenyl)ethanol is obtained. Furthermore, the purity of the resultant compound may be increased by distillation or purification by silica gel column chromatography or the like.

### Best Mode for Carrying Out the Invention

While the present invention will be described in more detail based on the examples below, it is to be understood that the invention is not limited thereto. Additionally, in the following description, "%" means %(W/V) unless stated otherwise.

### (EXAMPLE 1)

With respect to each of the microorganisms shown in Table 1, 50 ml of a culture medium (pH 7.0) comprising 1% of polypeptone, 1% of meat extract, 0.5% of yeast extract, and 0.3% of sodium chloride was placed into a 500-ml Sakaguchi flask, followed by sterilization, and the microorganism was inoculated into the culture medium. The microorganism was aerobically incubated in shake culture at 30°C for 2 days. The cells were collected from the culture medium by centrifugation and suspended in 5 ml of a 50 mM phosphate buffer (pH 6.5) containing 1% of 2-chloro-1-(3'-chlorophenyl)ethanone, 0.06% of oxidized nicotinamide adenine dinucleotide (NAD), 0.06% of oxidized nicotinamide adenine dinucleotide phosphate (NADP), 5% of glucose, and 14.3 U/ml of a glucose dehydrogenase (Trade name: "Amano 2" manufactured by Amano Enzyme Inc.). The resultant suspension was moved into a test tube. The test tube was plugged with cotton, and shaking was performed at 30°C for 24 hours. After the reaction, the reaction mixture was extracted with ethyl acetate in a volume two times that of the reaction mixture. The ethyl acetate layer was analyzed by high performance liquid chromatography, and the rate of reaction and the optical purity were measured. The results thereof are shown in Table 1.

**TABLE 1**

| Microorganisms | Rate of reaction (%) | Optical purity (%ee) | Configuration |
|---|---|---|---|
| Escherichia coli IFO 3301 | 2.9 | 73.9 | R |
| Escherichia coli IFO 13965 | 2.4 | 84.4 | R |
| Aerobacter aerogenes IFO 3166 | 1.3 | 10.1 | R |
| Enterobacter cloacae IFO 3320 | 7.2 | 12.3 | R |
| Klebsiella pneumoniae IFO 3318 | 5.1 | 67.1 | R |
| Klebsiella pneumoniae IFO 12059 | 4.3 | 53.9 | R |
| Citrobacter freundii IFO 13547 | 2.6 | 59.2 | R |
| Citrobacter freundii IFO 12681 | 4.0 | 75.2 | R |
| Rahnella aquatilis IFO 13544 | 2.7 | 82.5 | R |
| Erwinia carotovora subsp. carotovora IFO 3380 | 6.9 | 86.4 | R |
| Serratia marcescens IFO 3054 | 5.4 | 43.7 | R |
| Proteus mirabilis IFO 3849 | 3.4 | 12.1 | R |
| Morganella morganii subsp. morganii IFO 3848 | 27.6 | 36.3 | R |
| Salmonella typhimurium IFO 12529 | 1.7 | 22.6 | R |
| Salmonella typhimurium IFO 13245 | 2.4 | 40.8 | R |
| Alcaligenes faecalis IFO 13111 | 32.6 | 36.9 | R |
| Alcaligenes xylosoxidans subsp. denitrificans IFO 12669 | 9.5 | 21.4 | R |
| Kocuria rosea IFO 3764 | 30.3 | 6.7 | R |
| Arthrobacter pascens IFO 12139 | 45.8 | 90.7 | R |
| Arthrobacter protophormiae IFO 12128 | 29.9 | 77.3 | R |
| Brevibacterium linens IFO 12141 | 25.3 | 91.5 | R |
| Cellulomonas fimi IAM 12107 | 33.9 | 78.4 | R |
| Acinetobacter calcoaceticus IFO 12552 | 23.9 | 25.8 | R |
| Aeromonas hydrophila subsp. hydrophila IFO 3820 | 13.1 | 23.3 | R |
| Bacillus subtilis IFO 3009 | 3.8 | 21.6 | R |
| Bacillus thuringiensis IFO 3951 | 5.3 | 19.8 | R |
| Agrobacterium tumefaciens IFO 12667 | 2.0 | 13.2 | R |
| Nocardioides simplex IFO 12679 | 3.5 | 44.3 | R |
| Stenotrophomonas maltophilia IFO 12690 | 4.7 | 37.1 | R |
| Jensenia canicruria IFO 13914 | 77.4 | 42.6 | R |

### (EXAMPLE 2)

With respect to each of the microorganisms shown in Table 2, the same process was performed as in Example 1 except that a culture medium (pH 7.2) comprising 0.4% of glucose, 1.0% of malt extract, and 0.4% of yeast extract was used. The rate of reaction and the optical purity were measured. The results thereof are shown in Table 2.

**TABLE 2**

| Microorganisms | Rate of reaction (%) | Optical purity (%ee) | Configuration |
|---|---|---|---|
| Mycobacterium smegmatis IFO 3154 | 33.2 | 65.3 | R |
| Mycobacterium phlei IFO 3158 | 27.5 | 35.9 | R |
| Nocardia globerula IFO 13510 | 19.3 | 21.3 | R |
| Nocardia carnea IFO 14403 | 22.5 | 59.1 | R |
| Rhodococcus rhodochrous IFO 3338 | 27.3 | 87.2 | R |
| Rhodococcus erythropolis IFO 12320 | 32.0 | 91.6 | R |
| Pseudonocardia autotrophica IFO 12743 | 34.4 | 93.3 | R |
| Streptomyces griseolus IFO 3402 | 18.0 | 89.0 | R |
| Streptomyces scabies IFO 3111 | 26.7 | 67.2 | R |
| Streptomyces lactamdurans IFO 13305 | 12.6 | 45.9 | R |
| Streptosporangium roseum IFO 3776 | 2.7 | 52.3 | R |
| Rothia dentocariosa IFO 12531 | 3.4 | 12.5 | R |

### (EXAMPLE 3)

With respect to each of the microorganisms shown in Table 3, the same process was performed as in Example 1 except that a culture medium (pH 6.5) comprising 2% of malt extract, 2% of glucose, 0.3% of peptone, and 0.3% of yeast extract was used. The rate of reaction and the optical purity were measured. The results thereof are shown in Table 3.

**TABLE 3**

| Microorganisms | Rate of reaction (%) | Optical purity (%ee) | Configuration |
|---|---|---|---|
| Williopsis saturnus var. saturnus IFO 0125 | 7.8 | 80.1 | R |
| Kuraishia capsulata IFO 0974 | 20.8 | 56.6 | R |
| Citeromyces matritensis IFO 0954 | 12.1 | 67.9 | R |
| Saccharomycodes ludwigii IFO 1043 | 23.8 | 45.0 | R |
| Sporobolomyces salmonicolor IFO 1038 | 29.1 | 61.2 | R |
| Sporobolomyces roseus IFO 1106 | 20.5 | 38.9 | R |
| Dipodascus armillariae IFO 0102 | 4.4 | 79.3 | R |
| Dipodascus tetrasuperma CBS 765.70 | 4.9 | 92.4 | R |
| Saccharomycopsis capsularis IFO 0672 | 2.8 | 60.6 | R |
| Sporidiobolus johnsonii IFO 6903 | 30.7 | 14.6 | R |
| Zygosaccharomyces rouxii IFO 0493 | 10.5 | 72.6 | R |
| Hyphopichia burtonii IFO 0844 | 12.5 | 3.8 | R |
| Penicillium chrysogenus IFO 4626 | 5.7 | 39.4 | R |
| Penicillium oxalicum IFO 5748 | 4.2 | 27.0 | R |
| Penicillium rubrum IFO 6580 | 3.7 | 11.4 | R |
| Penicillium lilacinum IFO 5752 | 15.5 | 20.5 | R |
| Exophiala mansonii IFO 6880 | 29.7 | 34.5 | R |
| Sporotrichum aurantiacum IFO 9381 | 7.3 | 41.0 | R |
| Acremonium butyri IFO 8580 | 14.2 | 24.9 | R |
| Paecilomyces carneus IFO 8292 | 5.5 | 34.0 | R |
| Paecilomyces carneus IFO 8293 | 11.1 | 12.5 | R |
| Verticillium albo-atrum IFO 9470 | 23.8 | 13.7 | R |
| Verticillium dahliae IFO 9765 | 33.1 | 23.4 | R |
| Verticillium psalliotae IFO 30619 | 5.3 | 56.9 | R |
| Tilachlidium humicola IFO 5696 | 5.0 | 21.0 | R |
| Pitomyces chartarum ATCC 26953 | 2.1 | 19.1 | R |
| Monosporium bharatensis ATCC 18967 | 1.6 | 13.5 | R |
| Isaria japonica IFO 30367 | 5.8 | 45.0 | R |
| Gloeophyllum trabeum IFO 6509 | 11.2 | 29.5 | R |
| Strobilurus stephanocystis IFO 30194 | 5.4 | 27.8 | R |
| Crinipellis stipitaria IFO 30259 | 23.2 | 13.2 | R |

### (EXAMPLE 4)

50 ml of a culture medium (pH 7.0) comprising 1% of polypeptone, 1% of meat extract, 0.5% of yeast extract, and 0.3% of sodium chloride was placed into a 500-ml Sakaguchi flask, and sterilization was performed. Nocardia globerula IF013510 was inoculated into the culture medium. The flask was shaken at 30°C for 2 days aerobically to perform preculture. The culture medium (3,600 ml) was divided and poured into nine 2,000-ml Sakaguchi flasks, 400 ml each, and 5 ml of the culture broth was inoculated into each flask. Shake culture was performed aerobically at 30°C for 2 days. The cells were collected from the culture broth by centrifugation, suspended in 50 ml of a 100 mM phosphate buffer (pH 6.5), and disrupted with a SONIFIRE 250 ultrasonic homogenizer (manufactured by Branson Corp.). The fluid containing disrupted cell was stirred for 20 minutes in a hot water bath at 65°C, and the sediment was removed by centrifugation to prepare a crude enzyme solution. 1 g of 2-chloro-1-(3'-chlorophenyl)ethanone, 4 mg of oxidized nicotinamide adenine dinucleotide (NAD), 2 g of glucose, 600 U of a glucose dehydrogenase (Trade name: "Amano 2" manufactured by Amano Enzyme Inc.), and 2 ml of n-butyl acetate was added into the crude enzyme solution (18 ml), and stirring was performed at 30°C for 24 hours while maintaining the pH at 6.5 using a 5M sodium hydroxide aqueous solution. After the reaction was completed, the reaction mixture was extracted with ethyl acetate, and the solvent was removed. The extract was analyzed by high performance liquid chromatography, and the rate of reaction and the optical purity were measured. As a result, (R)-2-chloro-1-(3'-chlorophenyl)ethanol with an optical purity of 99.5% ee was produced at a yield of 46.9%.

### Industrial Applicability

According to the present invention, it becomes possible to produce optically active (R)-2-chloro-1-(3'-chlorophenyl)ethanol efficiently on an industrial scale. The resultant optically active (R)-2-chloro-1-(3'-chlorophenyl)ethanol is useful as a raw material for the synthesis of pharmaceuticals, etc.

## Claims

1. A process for producing (R)-2-chloro-1-(3'-chlorophenyl)ethanol comprising allowing a culture broth of a microorganism, cells of the microorganism, or a material prepared by treating the cells of the microorganism to act on 2-chloro-1-(3'-chlorophenyl)ethanone and collecting the resultant (R)-2-chloro-1-(3'-chlorophenyl)ethanol, the microorganism being capable of stereoselectively reducing 2-chloro-1-(3'-chlorophenyl)ethanone to yield (R)-2-chloro-1-(3'-chlorophenyl)ethanol and belonging to any one of the following genera: Escherichia, Aerobacter, Enterobacter, Klebsiella, Citrobacter, Rahnella, Erwinia, Serratia, Proteus, Morganella, Salmonella, Alcaligenes, Kocuria, Arthrobacter, Brevibacterium, Cellulomonas, Acinetobacter, Aeromonas, Bacillus, Agrobacterium, Nocardioides, Stenotrophomonas, Jensenia, Mycobacterium, Nocardia, Rhodococcus, Pseudonocardia, Streptomyces, Streptosporangium, Rothia, Williopsis, Kuraishia, Citeromyces, Saccharomycodes, Sporobolomyces, Dipodascus, Saccharomycopsis, Sporidiobolus, Zygosaccharomyces, Hyphopichia, Penicillium, Exophiala, Sporotrichum, Acremonium, Paecilomyces, Verticillium, Tilachlidium, Pitomyces, Monosporium, Isaria, Gloeophyllum, Strobilurus, and Crinipellis.

2. The process according to Claim 1, wherein the microorganism is any one of Escherichia coli, Aerobacter aerogenes, Enterobacter cloacae, Klebsiella pneumoniae, Citrobacter freundii, Rahnella aquatilis, Erwinia carotovora, Serratia marcescens, Proteus mirabilis, Morganella morganii, Salmonella typhimurium, Alcaligenes faecalis, Alcaligenes xylosoxidans, Kocuria rosea, Arthrobacter pascens, Arthrobacter protophormiae, Brevibacterium linens, Cellulomonas fimi, Acinetobacter calcoaceticus, Aeromonas hydrophila, Bacillus subtilis, Bacillus thuringiensis, Agrobacterium tumefaciens, Nocardioides simplex, Stenotrophomonas maltophilia, Jensenia canicruria, Mycobacterium smegmatis, Mycobacterium phlei, Nocardia globerula, Nocardia carnea, Rhodococcus rhodochrous, Rhodococcus erythropolis, Pseudonocardia autotrophica, Streptomyces griseolus, Streptomyces scabies, Streptomyces lactamdurans, Streptosporangium roseum, Rothia dentocariosa, Williopsis saturnus, Kuraishia capsulata, Citeromyces matritensis, Saccharomycodes ludwigii, Sporobolomyces salmonicolor, Sporobolomyces roseus, Dipodascus armillariae, Dipodascus tetrasuperma, Saccharomycopsis capsularis, Sporidiobolus johnsonii, Zygosaccharomyces rouxii, Hyphopichia burtonii, Penicillium chrysogenus, Penicillium oxalicum, Penicillium rubrum, Penicillium lilacinum, Exophiala mansonii, Sporotrichum aurantiacum, Acremonium butyri, Paecilomyces carneus, Verticillium albo-atrum, Verticillium dahliae, Verticillium psalliotae, Tilachlidium humicola, Pitomyces chartarum, Monosporium bharatensis, Isaria japonica, Gloeophyllum trabeum, Strobilurus stephanocystis, and Crinipellis stipitaria.
